# EUROPEAN PATENT SPECIFICATION

(11) **EP 2 280 974 B1**
(45) Date of publication and mention of the grant of the patent: **25.01.2012**
(21) Application number: 09739539.6
(22) Date of filing: 28.04.2009
(51) Int. Cl.: C07D 487/04, C07B 37/02, C07B 37/06, C07B 37/10, C07B 49/00, C07B 51/00, C07B 57/00

(54) **STEREOSELECTIVE SYNTHESIS OF CERTAIN TRIFLUOROMETHYL-SUBSTITUTED ALCOHOLS**
STEREOSELEKTIVE SYNTHESE BESTIMMTER TRIFLUORMETHYL-SUBSTITUIERTER ALKOHOLE
SYNTHÈSE STÉRÉOSÉLECTIVE DE CERTAINS ALCOOLS SUBSTITUÉS PAR TRIFLUOROMÉTHYLE

(30) Priority: 30.04.2008 US 49095 P
(43) Date of publication of application: 09.02.2011
(73) Proprietor: Boehringer Ingelheim International GmbH, 55216 Ingelheim (DE); Boehringer Ingelheim Pharma GmbH & Co. KG, 55216 Ingelheim (DE)
(72) Inventor: REEVES, Jonathan, Timothy, Ridgefield, CT 06877-0368 (US); SONG, Jinhua, J., Ridgefield, CT 06877-0368 (US); TAN, Zhulin, Ridgefield, CT 06877-0368 (US)
(74) Representative: Hammann, Heinz
(86) International application number: PCT/US2009/041883
(87) International publication number: WO 2009/134737

(56) References cited:
- WO-A-2007/040959
- US-A1- 2006 122 189
- R. M. SCHISLA, W. C. HAMMANN: "Quaternary-Substituted Hydrocarbons. A General Method of Synthesis of Hydrocarbons Interspersed with Four gem-Dimethyl Units" JOURNAL OF ORGANIC CHEMISTRY, vol. 35, no. 10, 1970, pages 3224-3230, XP002534782

## Description

### Field of the Invention

The present invention relates to an efficient stereoselective synthesis of certain trifluoromethyl-substituted alcohols.

### Background of the Invention

Trifluoromethyl-substituted alcohols of Formula (I) have been described as ligands that bind to the glucocorticoid receptor.

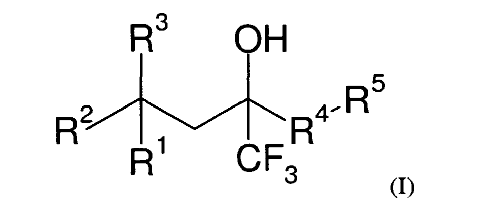

These compounds are effective as therapeutics in treating a number of diseases modulated by glucocorticoid receptor function, including inflammatory, autoimmune and allergic disorders. Examples of these compounds are described in U.S. Patent Nos. 7,268,152; 6,960,581; and 6,903,215, which are hereinafter termed "the Trifluoromethyl-Substituted Alcohol Patent Applications" .

It is well known in the art that enantiomers of a particular compound can have different biological properties including efficacy, toxicity, and pharmacokinetic properties. Thus, it is often desirable to administer one enantiomer of a racemic therapeutic compound.

The synthetic methods disclosed in the patent applications cited above describe the synthesis of racemic products. Separation of enantiomers was accomplished by chiral HPLC. Chiral HPLC and other enantiomer separation method, however, are generally unsuitable for large-scale preparation of a single enantiomer. Thus, a stereoselective synthesis for preparation of these compounds would be highly desirable.

WO 2007/40959 discloses a stereoselective synthesis for the preparation of these compounds.

The present invention discloses an efficient stereoselective synthesis of certain compounds of Formula (I). A key step involves an efficient chiral resolution of a beta-hydroxy acid and a one-step synthesis of a diazaindole subunit. The novel one-step azaindole synthesis from an ester has been previously described in our U.S. Serial No. 11/070,462. This new synthesis has fewer steps and utilizes relatively inexpensive starting materials, therefore providing a more economical synthesis of the drug substance.

### Summary of the Invention

The instant invention is directed to a process for stereoselective synthesis of a compound of Formula (I) 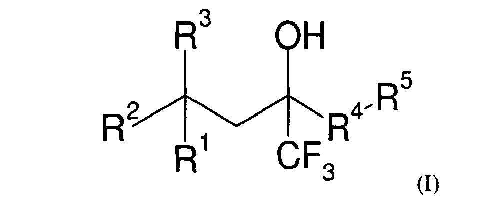 wherein:
- R¹: is an aryl or heteroaryl group, each optionally substituted with one to three substituent groups,
wherein each substituent group of R¹ is independently C₁-C₅ alkyl, *C₂-C₅* alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, C₁-C₅ alkanoyloxy, C₁-C₅ alkanoyl, aroyl, halogen, trifluoromethyl, trifluoromethoxy, or C₁-C5 alkylthio,
wherein each substituent group of R¹ is optionally independently substituted with one to three substituent groups selected from methyl, methoxy, fluoro, chloro, or alkoxy;
- R² and R³: are each independently hydrogen or C₁-C₅ alkyl, or R² and R³ together with the carbon atom they are commonly attached to form a C₃-C₈ spiro cycloalkyl ring;
- R⁴: is C₁-C₅ alkyl, C₂-C₅ alkenyl, or C₂-C₅ alkynyl, each optionally substituted with one to three substituent groups,
wherein each substituent group of R⁴ is independently C₁-C₃ alkyl, hydroxy, halogen, amino, or oxo; and
- R⁵: is the moiety 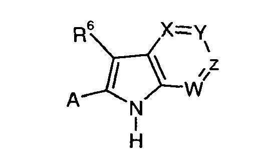
wherein A is the point of attachment to R⁴
W, X, Y, or Z is N or CH and at least two of W, X, Y, or Z is N, and
R⁶ is H, alkyl, or aryl, and
R⁵ is optionally substituted with one to three substituent groups, wherein each substituent group of R⁵ is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein each substituent group of R⁵ is optionally independently substituted with one to three substituent groups selected from C₁-C₃ alkyl, C₁-C₃ alkoxy, halogen, hydroxy, oxo, cyano, amino, or trifluoromethyl,
the process comprising:
(a) reacting a starting material of formula A with an unsaturated ester of formula B in the presence of a salt, without a solvent or with a suitable solvent, at a suitable temperature to provide an ester of formula C 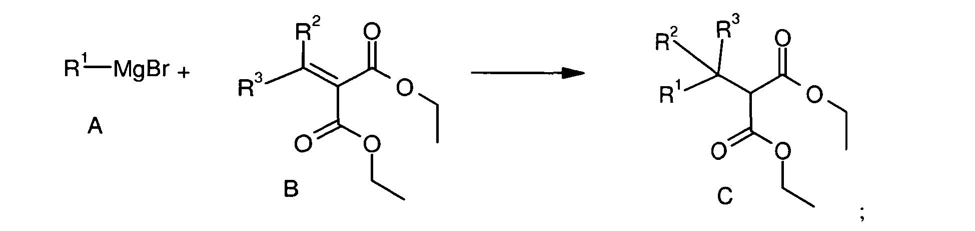
(b) hydrolyzing the ester of formula C using a suitable acid in water, with or without an organic solvent, at a suitable temperature to provide an acid of formula D 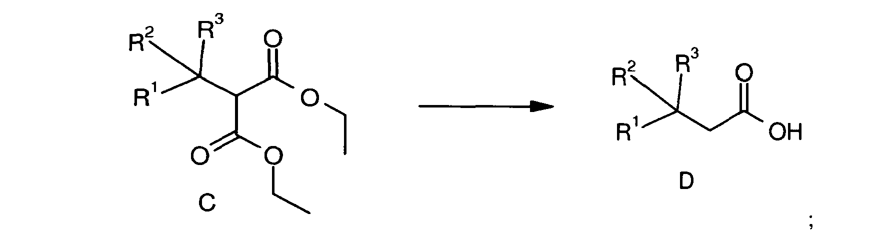
(c) reacting the acid of formula D with suitable trifluoroacetate in the presence of a suitable base in a suitable solvent at a suitable temperature to provide a trifluoromethyl ketone of formula E 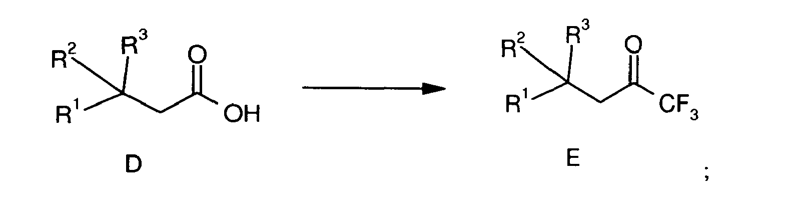
(d) reacting the trifluoromethyl ketone of formula E with an acetate in the presence of a suitable base in a suitable solvent at a suitable temperature to prepare an acid of formula F 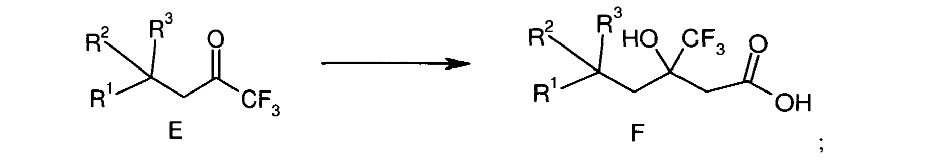
(e) reacting the acid of formula F with a suitable resolving base to provide a pure diastereomer followed by reacting the pure diastereomer with a suitable base in a suitable solvent at a suitable temperature to provide a pure enantiomer of formula G, or reacting a pure diastereomer of the acid of formula F with a suitable acid in a suitable solvent at a suitable temperature to obtain a pure enantiomer of formula G 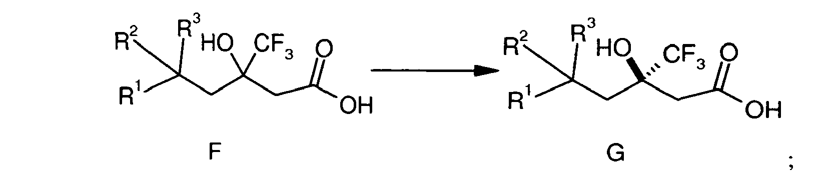
(f) reacting the acid of formula G with a suitable alcohol, R'-OH, where R' is an alkyl group, followed by protection of the tertiary alcohol with a protecting group agent PG-Y, where Y is a leaving group, at a suitable temperature to obtain the ester of formula H 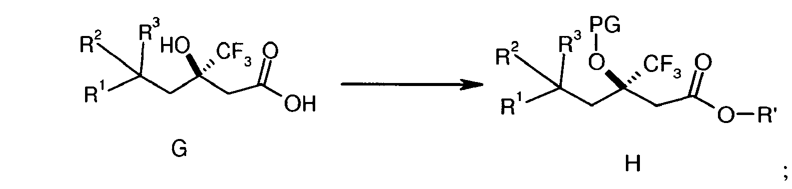 and
(g) reacting the ester of formula H with a compound of formula J, wherein W, X, Y, or Z is N or CH, at least two of W, X, Y, or Z is N, and R⁶ is H, alkyl, or aryl, in the presence of a suitable base in a suitable solvent at a suitable temperature to obtain a compound of Formula (I)

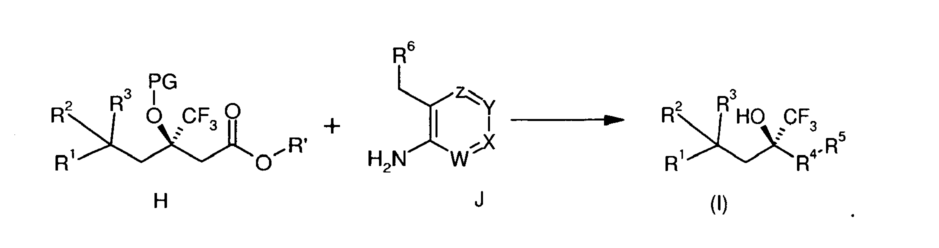

In an aspect of the invention, the suitable solvent of step (a) is tetrahydrofuran (THF), diethyl ether, dimethyl ether, dipropyl ether, diisopropyl ether, dibutyl ether, ethylene glycol dimethyl ether (DME), *tert*-butyl methyl ether (MTBE), or a mixture thereof, preferably THF. In another aspect of the invention, the suitable salt of step (a) is copper (I) chloride, copper (I) bromide, or copper (I) triflate, preferably copper (I) chloride. In another aspect of the invention, the suitable temperature of step (a) is 0°C to 30°C.

In an aspect of the invention, the suitable solvent of step (b) is water, alkyl alcohols such as MeOH, EtOH, or dimethyl ether, diethyl ether, dipropyl ether, diisopropyl ether, dibutyl ether, THF, DME, MTBE, or a mixture thereof, preferably water. In another aspect of the invention, the suitable acid of step (b) is acetic acid, sulfuric acid, hydrochloric acid, or a mixture thereof. In another aspect of the invention, the suitable temperature of step (b) is 0°C to 100°C.

In an aspect of the invention, the suitable solvent of step (c) is hydrocarbons such as toluene, xylene, heptane or alkyl ethers such as dimethyl ether, diethyl ether, dipropyl ether, diisopropyl ether, dibutyl ether, THF, DME, MTBE, or a mixture thereof, preferably toluene or xylene. In another aspect of the invention, the suitable base of step (c) is alkyl lithium, such as methyl lithium, *n*-BuLi, *sec*-BuLi, *tert*-BuLi, or LDA, lithium hexamethyldisilazide (LiHMDS), sodium hexamethyldisilazide (NaHMDS), or potassium hexamethyldisilazide (KHMDS), preferably LDA. In another aspect of the invention, the suitable temperature of step (c) is -50°C to 150°C.

In an aspect of the invention, the suitable solvent of step (d) is THF, DME, diethyl ether, dipropyl ether, diisopropyl ether, dibutyl ether, MTBE, toluene, xylene, or dimethylformamide (DMF). In another aspect of the invention, the suitable base of step (d) is LiHMDS, NaHMDS, KHMDS, LDA, LiH, NaH, KH, or NaNH₂. In another aspect of the invention, the suitable acetate reagent of step (d) is methyl acetate, ethyl acetate, propyl acetate, or butyl acetate. In another aspect of the invention, the suitable temperature of step (d) is -70°C to 50°C.

In yet another aspect of the invention, the suitable resolving base of step (e) is (+ or -) *cis-*1-amino-2-indanol, quinine, quinidine, (+ or -) ephedrine, (+ or -) deoxyephedrine, (+ or -) methylbenzylamine, (+ or -) (1-naphthyl)ethylamine, or (+ or -) (2-naphthyl)ethylamine. In another aspect of the invention, the suitable base of step (e) is potassium hydroxide, lithium hydroxide, sodium bicarbonate, sodium carbonate, potassium bicarbonate, potassium carbonate, or lithium carbonate. Alternatively, a pure enantiomer of formula G is obtained by reacting a pure diastereomer with a suitable acid, such as hydrochloric acid or sulfuric acid. In another aspect of the invention, the suitable solvent of step (e) is dichloromethane, diethyl ether, ethyl acetate, isopropyl acetate, *n*-butyl acetate, heptane, hexane, toluene, xylene, MTBE, or a mixture thereof, preferably a mixture of heptane and *n*-butyl acetate. In another aspect of the invention, the suitable temperature of step (e) is -40°C to 150°C.

In another aspect of the invention, the esterification of step (f) is carried out by treatment of the free acid with a lower alkyl alcohol such as methanol or ethanol in the presence of an acid catalyst selected from sulfuric acid, hydrochloric acid, acetic acid, *p*-toluenesulfonic acid, and acetic acid. In another aspect of the invention, in the protecting group agent PG-Y of step (f), the protecting group PG is a trialkylsilyl group, lower alkyl ether (e.g., methoxymethyl ether (MOM ether)), lower alkyl group, or internally protected as a β-lactone with the terminal carboxyl group, and the leaving group Y is Cl, Br, I, MsO, TsO, and TfO, preferably Cl. In another aspect of the invention, the suitable solvent of step (f) is dichloromethane, DMF, THF, diethyl ether, dipropyl ether, diisopropyl ether, dibutyl ether, DME, ethyl acetate, isopropyl acetate, *n*-butyl acetate, heptane, hexane, toluene, xylene, MTBE, or a mixture thereof, preferably DMF. In another aspect of the invention, the suitable temperature of step (f) is -70°C to 150°C.

In another aspect of the invention, the suitable solvent of step (g) is THF, DME, diethyl ether, dipropyl ether, diisopropyl ether, dibutyl ether, MTBE, toluene, benzene, xylene, hexane, pentane, heptane, methylene chloride, or a mixture thereof, and is preferably THF. In another aspect of the invention, the suitable base of step (g) is *n*-BuLi, *sec*-BuLi, *tert-*BuLi, or LDA, optionally including additives such as *N,N,N',N'-*tetramethylethylenediamine (TMEDA), β-dialkylaminoalcohols, sparteine, or polyethers, preferably *sec-*BuLi. In another aspect of the invention, the suitable temperature of step (g) is -70°C to 80°C.

In another aspect of the invention, the compound of formula J is 4-methyl-5-aminopyrimidine, 4-amino-5-methylpyrimidine, 5-amino-6-methylpyrimidine, or 5-methyl-6-aminopyrimidine, each optionally substituted on the ring or methyl group with a substituent compatible with alkyl lithium, preferably 4-methyl-5-aminopyrimidine.

While certain specific embodiments of the invention, including specific reaction conditions, solvents, protecting groups, and other reagents and reactants are described above in detailing various aspects of the invention, it should be understood that no particular limitation to these specific embodiments or aspects should limit the invention in its broadest sense. Accordingly, the invention should be understood to include none, some, or all of these various aspects in various combinations.

### Detailed Description of the Invention

### Definition of Terms and Conventions Used

Terms not specifically defined herein should be given the meanings that would be given to them by one of skill in the art in light of the disclosure and the context. As used in the specification and appended claims, however, unless specified to the contrary, the following terms have the meaning indicated and the following conventions are adhered to.

### A. Chemical Nomenclature, Terms, and Conventions

In the groups, radicals, or moieties defined below, the number of carbon atoms is often specified preceding the group, for example, C₁-C₁₀ alkyl means an alkyl group or radical having 1 to 10 carbon atoms. The term "lower'' applied to any carbon-containing group means a group containing from 1 to 8 carbon atoms, as appropriate to the group (i.e., a cyclic group must have at least 3 atoms to constitute a ring). In general, for groups comprising two or more subgroups, the last named group is the radical attachment point, for example, "alkylaryl" means a monovalent radical of the formula Alk-Ar-, while "arylalkyl" means a monovalent radical of the formula Ar-Alk- (where Alk is an alkyl group and Ar is an aryl group). Furthermore, the use of a term designating a monovalent radical where a divalent radical is appropriate shall be construed to designate the respective divalent radical and *vice versa.* Unless otherwise specified, conventional definitions of terms control and conventional stable atom valences are presumed and achieved in all formulas and groups.

The terms "alkyl" or "alkyl group" mean a branched or straight-chain saturated aliphatic hydrocarbon monovalent radical. This term is exemplified by groups such as methyl, ethyl, *n-*propyl, 1-methylethyl (isopropyl), *n-*butyl, *n-*pentyl, 1,1-dimethylethyl (*tert*-butyl), and the like. It may be abbreviated "Alk".

The terms "alkenyl" or "alkenyl group" mean a branched or straight-chain aliphatic hydrocarbon monovalent radical containing at least one carbon-carbon double bond. This term is exemplified by groups such as ethenyl, propenyl, *n-*butenyl, isobutenyl, 3-methylbut-2-enyl, *n-*pentenyl, heptenyl, octenyl, decenyl, and the like.

The terms "alkynyl" or "alkynyl group" mean a branched or straight-chain aliphatic hydrocarbon monovalent radical containing at least one carbon-carbon triple bond. This term is exemplified by groups such as ethynyl, propynyl, *n-*butynyl, 2-butynyl, 3-methylbutynyl, *n-*pentynyl, heptynyl, octynyl, decynyl, and the like.

The terms "alkylene" or "alkylene group" mean a branched or straight-chain saturated aliphatic hydrocarbon divalent radical having the specified number of carbon atoms. This term is exemplified by groups such as methylene, ethylene, propylene, *n*-butylene, and the like, and may alternatively and equivalently be denoted herein as -(alkyl)-.

The terms "alkenylene" or "alkenylene group" mean a branched or straight-chain aliphatic hydrocarbon divalent radical having the specified number of carbon atoms and at least one carbon-carbon double bond. This term is exemplified by groups such as ethenylene, propenylene, *n-*butenylene, and the like, and may alternatively and equivalently be denoted herein as -(alkylenyl)-.

The terms "alkynylene" or "alkynylene group" mean a branched or straight-chain aliphatic hydrocarbon divalent radical containing at least one carbon-carbon triple bond. This term is exemplified by groups such as ethynylene, propynylene, *n-*butynylene, 2-butynylene, 3-methylbutynylene, *n-*pentynylene, heptynylene, octynylene, decynylene, and the like, and may alternatively and equivalently be denoted herein as -(alkynyl)-.

The terms "alkoxy" or "alkoxy group" mean a monovalent radical of the formula AlkO-, where Alk is an alkyl group. This term is exemplified by groups such as methoxy, ethoxy, propoxy, isopropoxy, butoxy, *sec*-butoxy, *tert-*butoxy, pentoxy, and the like.

The terms "aryloxy", "aryloxy group", mean a monovalent radical of the formula ArO-, where Ar is aryl. This term is exemplified by groups such as phenoxy, naphthoxy, and the like.

The terms "alkylcarbonyl", "alkylcarbonyl group", "alkanoyl", or "alkanoyl group" mean a monovalent radical of the formula AlkC(O)-, where Alk is alkyl or hydrogen.

The terms "arylcarbonyl", "arylcarbonyl group", "aroyl" or "aroyl group" mean a monovalent radical of the formula ArC(O)-, where Ar is aryl.

The terms "acyl" or "acyl group" mean a monovalent radical of the formula RC(O)-, where R is a substituent selected from hydrogen or an organic substituent. Exemplary substituents include alkyl, aryl, arylalkyl, cycloalkyl, heterocyclyl, heteroaryl, heteroarylalkyl, and the like. As such, the terms comprise alkylcarbonyl groups and arylcarbonyl groups.

The terms "acylamino'' or "acylamino group" mean a monovalent radical of the formula RC(O)N(R)-, where each R is a substituent selected from hydrogen or a substituent group.

The terms "alkoxycarbonyl" or "alkoxycarbonyl group" mean a monovalent radical of the formula AlkO-C(O)-, where Alk is alkyl. Exemplary alkoxycarbonyl groups include methoxycarbonyl, ethoxycarbonyl, *tert-*butyloxycarbonyl, and the like.

The terms "alkylaminocarbonyloxy" or "alkylaminocarbonyloxy group" mean a monovalent radical of the formula R₂NC(O)O-, where each R is independently hydrogen or lower alkyl.

The term "alkoxycarbonylamino" or "alkoxycarbonylamino group" mean a monovalent radical of the formula ROC(O)NH-, where R is lower alkyl.

The terms "alkylcarbonylamino" or "alkylcarbonylamino group" or "alkanoylamino" or "alkanoylamino groups" mean a monovalent radical of the formula AlkC(O)NH-, where Alk is alkyl. Exemplary alkylcarbonylamino groups include acetamido (CH₃C(O)NH-).

The terms "alkylaminocarbonyloxy" or "alkylaminocarbonyloxy group" mean a monovalent radical of the formula AlkNHC(O)O-, where Alk is alkyl.

The terms "amino" or "amino group" mean an -NH₂ group.

The terms "alkylamino" or "alkylamino group" mean a monovalent radical of the formula (Alk)NH-, where Alk is alkyl. Exemplary alkylamino groups include methylamino, ethylamino, propylamino, butylamino, *tert*-butylamino, and the like.

The terms "dialkylamino" or "dialkylamino group" mean a monovalent radical of the formula (Alk)(Alk)N-, where each Alk is independently alkyl. Exemplary dialkylamino groups include dimethylamino, methylethylamino, diethylamino, dipropylamino, ethylpropylamino, and the like.

The terms "substituted amino" or "substituted amino group" mean a monovalent radical of the formula -NR₂, where each R is independently a substituent selected from hydrogen or the specified substituents (but where both Rs cannot be hydrogen). Exemplary substituents include alkyl, alkanoyl, aryl, arylalkyl, cycloalkyl, heterocyclyl, heteroaryl, heteroarylalkyl, and the like.

The terms "alkoxycarbonylamino" or "alkoxycarbonylamino group" mean a monovalent radical of the formula AlkOC(O)NH-, where Alk is alkyl.

The terms "ureido" or "ureido group" mean a monovalent radical of the formula R₂NC(O)NH-, where each R is independently hydrogen or alkyl.

The terms "halogen" or "halogen group" mean a fluoro, chloro, bromo, or iodo group.

The term "halo" means one or more hydrogen atoms of the group are replaced by halogen groups.

The terms "alkylthio" or "alkylthio group" mean a monovalent radical of the formula AlkS-, where Alk is alkyl. Exemplary groups include methylthio, ethylthio, *n-*propylthio, isopropylthio, *n-*butylthio, and the like.

The terms "sulfonyl" or "sulfonyl group" mean a divalent radical of the formula -SO₂-.

The terms "carbocycle" or "carbocyclic group" mean a stable aliphatic 3- to 15-membered monocyclic or polycyclic monovalent or divalent radical consisting solely of carbon and hydrogen atoms which may comprise one or more fused or bridged ring(s), preferably a 5-to 7-membered monocyclic or 7- to 10-membered bicyclic ring. Unless otherwise specified, the carbocycle may be attached at any carbon atom which results in a stable structure and, if substituted, may be substituted at any suitable carbon atom which results in a stable structure. The term comprises cycloalkyl (including spiro cycloalkyl), cycloalkylene, cycloalkenyl, cycloalkenylene, cycloalkynyl, and cycloalkynylene, and the like.

The terms "cycloalkyl" or "cycloalkyl group" mean a stable aliphatic saturated 3- to 15-membered monocyclic or polycyclic monovalent radical consisting solely of carbon and hydrogen atoms which may comprise one or more fused or bridged ring(s), preferably a 5-to 7-membered monocyclic or 7- to 10-membered bicyclic ring. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornanyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

The terms "aryl" or "aryl group" mean an aromatic carbocyclic monovalent or divalent radical of from 6 to 14 carbon atoms having a single ring (e.g., phenyl or phenylene) or multiple condensed rings (e.g., naphthyl or anthranyl). Unless otherwise specified, the aryl ring may be attached at any suitable carbon atom which results in a stable structure and, if substituted, may be substituted at any suitable carbon atom which results in a stable structure. Exemplary aryl groups include phenyl, naphthyl, anthryl, phenanthryl, indanyl, indenyl, biphenyl, and the like. It may be abbreviated "Ar".

The terms "heteroaryl" or "heteroaryl group" mean a stable aromatic 5- to 14-membered, monocyclic or polycyclic monovalent or divalent radical which may comprise one or more fused or bridged ring(s), preferably a 5- to 7-membered monocyclic or 7- to 10-membered bicyclic radical, having from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Exemplary and preferred heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrinidinyl, furanopyrazinyl, furanopyridazinyl, dihydrobenzofuranyl, dihydrofuranopyridinyl, dihydrofuranopyrimidinyl, benzodioxolanyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl, and the like.

The terms "heterocycle", "heterocycle group", "heterocyclyl", or "heterocyclyl group" mean a stable non-aromatic 5- to 14-membered monocyclic or polycyclic, monovalent or divalent, ring which may comprise one or more fused or bridged ring(s), preferably a 5- to 7-membered monocyclic or 7- to 10-membered bicyclic ring, having from one to three heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heterocyclyl ring may be attached at any suitable heteroatom or carbon atom which results in a stable structure and, if substituted, may be substituted at any suitable heteroatom or carbon atom which results in a stable structure. Exemplary and preferred heterocycles include pyrrolinyl, pyrrolidinyl, pyrazolinyl, pyrazolidinyl, piperidinyl, morpholinyl, thiomorpholinyl, piperazinyl, tetrahydropyranyl, tetrahydrothiopyranyl, tetrahydrofuranyl, hexahydropyrimidinyl, hexahydropyridazinyl, and the like.

The term "compounds of the invention" and equivalent expressions are meant to embrace compounds of Formula (I) as herein described, including the tautomers, the salts, particularly the pharmaceutically acceptable salts, and the solvates and hydrates thereof, where the context so permits. In general and preferably, the compounds of the invention and the formulas designating the compounds of the invention are understood to only include the stable compounds thereof and exclude unstable compounds, even if an unstable compound might be considered to be literally embraced by the compound formula. Similarly, reference to intermediates, whether or not they themselves are claimed, is meant to embrace their salts and solvates, where the context so permits. For the sake of clarity, particular instances when the context so permits are sometimes indicated in the text, but these instances are purely illustrative and it is not intended to exclude other instances when the context so permits.

The term "leaving group" means a group with the meaning conventionally associated with it in synthetic organic chemistry, that is, an atom or group displaceable under substitution reaction conditions. Examples of leaving groups include, but are not limited to, halogen, alkane- or arylenesulfonyloxy, such as methanesulfonyloxy, ethanesulfonyloxy, thiomethyl, benzenesulfonyloxy, tosyloxy, and thienyloxy, dihalophosphinoyloxy, optionally substituted benzyloxy, isopropyloxy, acyloxy, and the like.

The term "solvent" or "suitable solvent" means a solvent or a mixture of solvents that is substantially inert under the conditions of the reaction being described in conjunction therewith, including, for example, dimethyl ether, diethyl ether, dipropyl ether, diisopropyl ether, dibutyl ether, tetrahydrofuran (THF), ethylene glycol dimethyl ether (DME), *tert-*butyl methyl ether (MTBE), benzene, toluene, acetonitrile, *N,N*-dimethylformamide, chloroform, methylene chloride, dichloroethane, ethyl acetate, acetone, methyl ethyl ketone, methanol, ethanol, propanol, isopropanol, *tert-*butanol, dioxane, pyridine, and the like, or mixtures thereof. Unless specified to the contrary, the solvents used in the reactions of the present invention are substantially inert solvents.

The term "protecting group" means a chemical group which selectively blocks one reactive site in a multifunctional compound such that a chemical reaction can be carried out selectively at another unprotected reactive site in the meaning conventionally associated with it in synthetic chemistry. Certain synthetic steps of the invention rely upon the protective groups to block reactive atoms, for example, nitrogen or hydrogen atoms, present in the reactants. For example, an amino protecting group or nitrogen protecting group is an organic group intended to protect the nitrogen atom against undesirable reactions during synthetic procedures. Exemplary nitrogen protecting groups include, but are not limited to, trifluoroacetyl, acetamido, benzyl (Bn), benzyloxycarbonyl (carbobenzyloxy, CBZ), *p-*methoxybenzyloxycarbonyl, *p-*nitrobenzyloxycarbonyl, *tert-*butoxycarbonyl (BOC), and the like. Similarly, a hydroxy protecting group is an organic group intended to protect the oxygen atom of a hydroxyl group against undesirable reactions during synthetic procedures. Exemplary hydroxy protecting groups include, but are not limited to benzyl, silyl groups, tetrahydropyranyl, esters, and the like. One of skill in the art, based on the instant specification, will know how to chose a suitable protecting group for the ease of removal and for the ability to withstand the subsequent reactions. Certain protecting groups are described, for example, in J.F.W. McOmie (ed.), Protective Groups in Organic Chemise, Plenum Press, 1973; T.W. Greene & P.G.M. Wuts, Protective Groups in Organic Synthesis (3rd Ed.), John Wiley & Sons, 1999; and P.J. Kocienski, Protecting Groups (2nd Ed.) Theime Medical Pub., 2000. Protecting groups may be removed at a convenient subsequent stage using methods known in the art or by metabolic or other *in vivo* administration conditions.

The term "protecting group agent" means reaction conditions or a reagent that supplies a desired protecting group to the substrate.

The terms "optional" or "optionally" mean that the subsequently described event or circumstances may or may not occur, and that the description includes instances where the event or circumstance occurs and instances in which it does not. For example, "optionally substituted aryl" means that the aryl radical may or may not be substituted and that the description includes both substituted aryl radicals and aryl radicals having no substitution.

The terms "stable compound" or "stable structure" mean a compound that is sufficiently robust to survive isolation to a useful degree of purity from a reaction mixture, and formulation into an efficacious therapeutic or diagnostic agent. For example, a compound which would have a "dangling valency" or is a carbanion is not a compound contemplated by the invention.

The term "substituted" means that any one or more hydrogens on an atom of a group or moiety, whether specifically designated or not, is replaced with a selection from the indicated group of substituents, provided that the atom's normal valency is not exceeded and that the substitution results in a stable compound. If a bond to a substituent is shown to cross the bond connecting two atoms in a ring, then such substituent may be bonded to any atom on the ring. When a substituent is listed without indicating the atom via which such substituent is bonded to the rest of the compound, then such substituent may be bonded via any atom in such substituent. For example, when the substituent is piperazinyl, piperidinyl, or tetrazolyl, unless specified otherwise, such piperazinyl, piperidinyl, or tetrazolyl group may be bonded to the rest of the compound of the invention via any atom in such piperazinyl, piperidinyl, or tetrazolyl group. Generally, when any substituent or group occurs more than one time in any constituent or compound, its definition on each occurrence is independent of its definition at every other occurrence. Thus, for example, if a group is shown to be substituted with 0 to 2 R⁵, then such group is optionally substituted with up to two R⁵ groups and R⁵ at each occurrence is selected independently from the defined list of possible R⁵. Such combinations of substituents and/or variables, however, are permissible only if such combinations result in stable compounds.

In a specific embodiment, the term "about" or "approximately" means within 20%, preferably within 10%, and more preferably within 5% of a given value or range.

The yield of each of the reactions described herein is expressed as a percentage of the theoretical yield.

### Experimental Example

The invention provides processes for making compounds of Formula (I). In all schemes, unless specified otherwise, R¹ to R⁵ in the formulas below can have the meanings of R¹ to R⁵ set forth herein. Intermediates used in the preparation of compounds of the invention are either commercially available or readily prepared by methods known to those skilled in the art.

Optimum reaction conditions and reaction times may vary depending on the particular reactants used. Unless otherwise specified, solvents, temperatures, pressures, and other reaction conditions may be readily selected by one of ordinary skill in the art. Specific procedures are provided in the Experimental Example section. Typically, reaction progress may be monitored by high performance liquid chromatography (HPLC) or thin layer chromatography (TLC), if desired, and intermediates and products may be purified by chromatography on silica gel and/or by recrystallization.

### Synthetic Example

The following is a representative example that illustrates the process of the invention. HPLC used to characterize products and intermediates were done on a C₁₈ Super-ODS column (Supelco, part No. 818197, 4.6 mm x 10 cm) eluting with a gradient of 5% acetonitrile/95% waster/0.05% TFA to 95% acetonitrile/5% water/0.05% TFA over 15 minutes and then held at 95% acetonitrile/5% water/0.05% TFA for 5 minutes. References to concentration or evaporation of solutions refer to concentration on a rotary evaporator.

### Example: Synthesis of (R)-1,1,1-Trifluoro-4-(5-fluoro-2-methylphenyl)-4-methyl-2-(5H-pyrrolo[3,2-d]pyrimidin-6-ylmethyl)pentan-2-ol

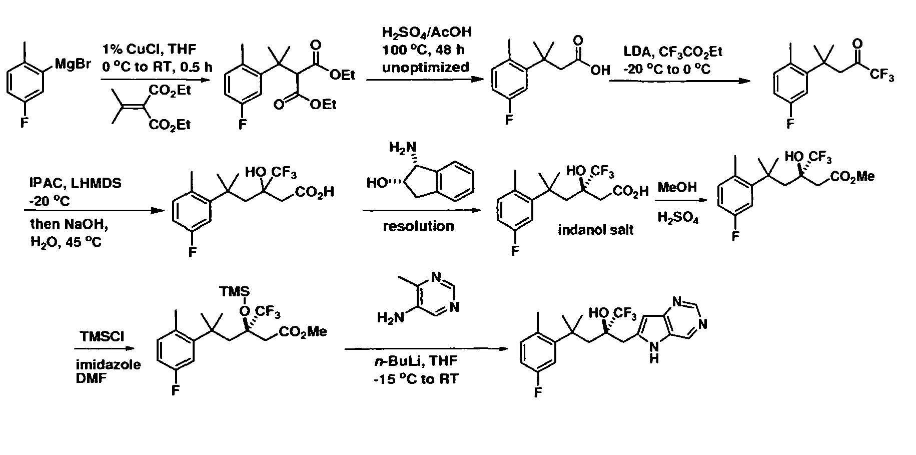

The details of the synthesis are as follows.

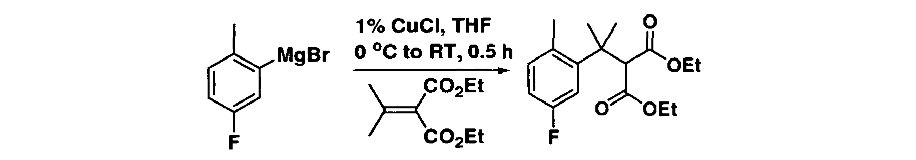

### Stage 1:

0.10 g of copper (I) chloride was added to a 500 mL flask and the system was purged with nitrogen. 107.0 mL of 5-fluoro-2-methylphenylmagnesium bromide (1.0 M in THF) was added via cannulation. The batch temperature was set to 0°C ± 2°C. 20.0 mL of diethyl isopropylidene malonate was added at a rate to maintain the batch temperature between 0°C to 15°C (approximately 20 minutes). The reaction mixture was aged for 30 minutes at 0°C to 15°C. The batch was cooled to 0°C ± 5°C. A solution of 25 mL of concentrated HCl in 25 mL of water was added at a rate to maintain the batch temperature below 35°C. The mixture was agitated vigorously until the batch temperature reached 20°C ± 5°C. Layers were separated and the organic phase was concentrated to remove THF. 36.1 g of crude 2-[1-(5-fluoro-2-methylphenyl)-1-methylethyl]malonic acid diethyl ester was obtained as a light green oil which was used directly without additional purification in Stage 2.

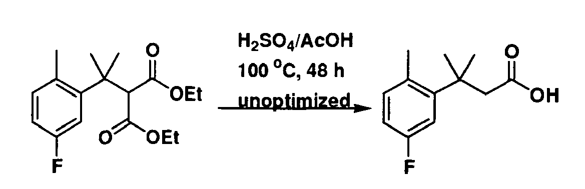

### Stage 2:

1.60 kg of crude malonic diethyl ester from Stage 1 was added to a 22 L flask followed by 4.5 L acetic acid. A solution of concentrated sulfuric acid (0.5 L) and water (2.5 L) were added, and the batch temperature was set to 100°C ± 5°C. The batch was kept at 100°C ± 5°C (reflux) for 48 hours. 750 mL was distilled from the reaction mixture (the batch temperature was maintained at 100°C ± 5°C during the distillation). The batch was cooled to 20°C ± 5°C. 4.0 L of water was added with vigorous agitation. Approximately 3 g of seed crystals were introduced. The mixture was agitated for 1 to 2 hours at 20°C ± 5°C. The solid was filtered and washed with 4.0 L water followed by 1.0 L ice-cold heptane. The product was dried *in vacuo* at 55°C with a nitrogen sweep until water content was <0.2%. The yield was 766 g of 3-(5-fluoro-2-methylphenyl)-3-methylbutyric acid as a tan solid (80% yield over two stages) with an HPLC purity of 98.0 A% (220 nm).

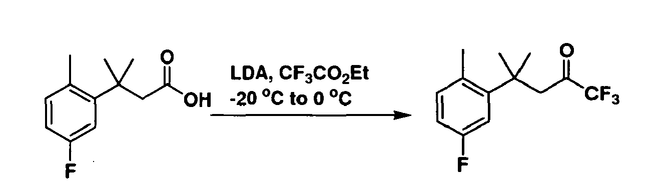

### Stage 3:

An aqueous solution of sodium hydroxide was prepared by dissolving 150.0 g of sodium hydroxide into 900 mL of water. An aqueous solution of sodium chloride was prepared by dissolving 30.0 g of sodium chloride in 900 mL of water. 3-(5-Fluoro-2-methylphenyl)-3-methylbutyric acid from the above reaction (300.0 g) was added to the reactor under nitrogen atmosphere followed by THF (533.4 g, 600.0 mL). The solution was cooled to an internal temperature of -20°C ± 3°C. With agitation of 300-500 rpm, LDA mono(THF) solution (1.40 kg) was added at a rate that maintained the internal temperature below -5°C. The addition required 65 minutes. The internal temperature was ramped to 10°C-15°C over 30 minutes. The reaction mixture was held at 10°C to no more than 15°C for 2 to 4 hours (agitation at 200-300 rpm). THF (800.4 g, 900.0 mL) and ethyl trifluoroacetate (608.4 g, 509.0 mL) were mixed in a second reactor under nitrogen atmosphere. The solution was cooled to an internal temperature of -20°C ± 3°C. The enolate solution from the first reactor was added into the second reactor at a rate that internal temperature was no more than -17°C. 15 minutes after addition was complete, concentrated hydrochloric acid (408.0 g, 390.0 mL) was added at a rate that internal temperature did not exceed 25°C. A thick orange-yellow slurry was obtained with pH between 2 and 4. The batch was maintained at 15°C-25°C for no less than 1 hour followed by addition of water (450 mL). The internal temperature rose from 21°C to 22°C. The reaction mixture was agitated for 5 to 10 minutes, the layers were allowed to settle, and the lower aqueous phase was drained. The sodium hydroxide solution (1050.0 g) was then added. The mixture was agitated for 5 to 10 minutes and the layers were separated. The sodium chloride solution (930.0 g) was added. The mixture was agitated for 5 to 10 minutes and the lower aqueous phase was drained. The majority of THF and cyclohexane was distilled off at atmospheric pressure. 269.6 g of a red-orange solution was obtained which was 71.7 wt. % 1,1,1-trifluoro-4-(5-fluoro-2-methylphenyl)-4-methylpentan-2-one by wt.% assay (193.3 g product, 51.7% yield).

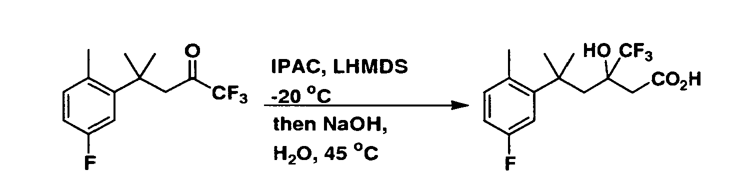

### Stage 4:

An aqueous solution of sodium hydroxide was prepared by dissolving sodium hydroxide (14.9 g) in water (120 g, 120 mL). An aqueous solution of hydrochloric acid was prepared by adding 65.0 mL of concentrated hydrochloric acid to 65.0 mL of water. A solution of 1,1,1-trifluoro-4-(5-fluoro-2-methylphenyl)-4-methylpentan-2-one (50.0 g), isopropyl acetate (38.0 g, 43.6 mL), and THF (38.8 g, 43.6 mL) was prepared. LIHMDS, solution (355.0 mL) was added to the reactor under a nitrogen atmosphere. The solution was cooled to an internal temperature of -20°C ± 3°C. The methyl pentanone solution from Step 3 was added at a rate that the internal temperature was kept below -17°C. The batch was kept at an internal temperature of -20°C to -15°C. 15 minutes after addition of the methyl pentanone solution was complete, the sodium hydroxide solution (134.9 g) was added. The external temperature was ramped to 70°C ± 3°C over 30 minutes and held at this temperature for no less than 3 hours (gentle reflux). The external temperature was ramped to 70°C-82°C and at least 350 mL of THF was distilled from the reaction mixture. The reaction mixture was cooled to 20°C ± 5°C and water (100 mL) and methanol (59.3 g, 75 mL) were added. The mixture was agitated for 5 to 10 minutes. Heptane (100 mL) was added. The mixture was agitated for 5 to 10 minutes and the layers were cut. The aqueous phase was returned to the reactor. The heptane wash was repeated two more times. The aqueous 6 N HCl solution (143 g, 130 mL) was added at a rate that internal temperature did not exceed 25°C. Seed crystals (-50 mg) were added to the reaction mixture. The mixture was agitated vigorously for no less than 1 hour at 20°C ± 5°C. The solid (fast filter time) was filtered and washed with two 100 mL portions of water. This was transferred to a drying oven and dried at 55°C for 16 hours. 54.18 g of 5-(5-fluoro-2-methylphenyl)-3-hydroxy-5-methyl-3-tritluoromethylhexanoic acid was obtained with HPLC purity of 91.9% (84.4% yield).

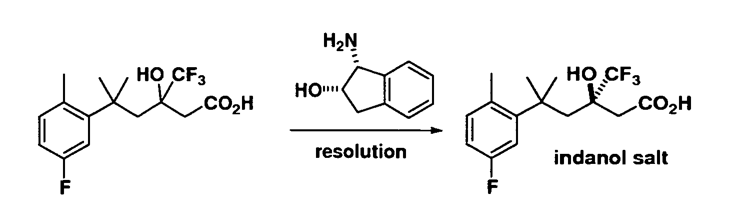

### Stage 5:

A solution of *n*-BuOAc/heptane was prepared by mixing 920 mL of *n*-BuOAc and 980 mL of heptane. 900.0 g of 5-(5-fluoro-2-methylphenyl)-3-hydroxy-5-methyl-3-trifluoromethylhexanoic acid was added to a reactor followed by 404.1 g of (1*R*,2*S*)-(+)-*cis-*1-amino-2-indanol. 3.04 L of *n*-BuOAc was added to the reactor. The mixture was heated to an internal temperature of 90°C-93°C and held at this temperature for about 15 minutes and cooled to 10°C linearly over 3 hours. It was held at 10°C-12°C for 3 hours. The solid was filtered and washed with the *n-*BuOAc/heptane solution (1.9 L, 1479.0 g) and finally with 1.35 L of heptane. It was dried at 60°C under vacuum for 4 hours to give 580.0 g of (*R*)-5-(5-fluoro-2-methylphenyl)-3-hydroxy-5-methyl-3-trifluoromethylhexanoic acid as a white solid (45.4% yield) with 97.7% ee.

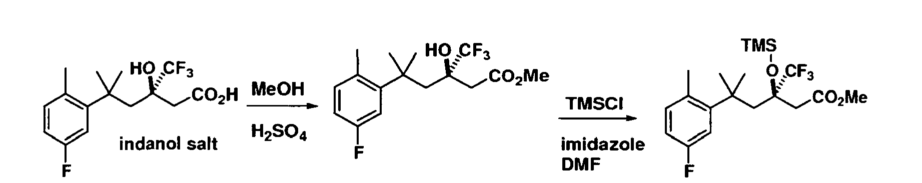

### Stages 6 & 7:

An aqueous solution of sodium chloride was prepared by dissolving 5.0 g of NaCl in 250 mL of water. A first aqueous solution of sodium bicarbonate was prepared by dissolving 5.0 g of sodium bicarbonate in 250 mL of water. A solution of imidazole in DMF was prepared by dissolving 72.2 g of imidazole in 175 mL of DMF. An aqueous solution of sodium bicarbonate was prepared by dissolving 10.0 g of sodium bicarbonate in 300 mL of water (note: this second sodium bicarbonate solution was for use in stage 2 of the process.). (R)-5-(5-Fluoro-2-methylphenyl)-3-hydroxy-5-methyl-3-trifluoromethylhexanoic acid (200.0 g) was added to the reactor followed by methanol. Concentrated sulfuric acid was added over approximately 2 minutes, keeping internal temperature below 35°C. The jacket temperature was ramped to 65°C over 30 minutes to achieve a gentle reflux. The batch was held at 65°C for no less than 15 hours and cooled to 15°C-20°C. Heptane (600 mL) and water (400 mL) were added. The batch was agitated for 10 minutes and the layers were cut. The aqueous phase was extracted with heptane (300 mL) again. To the combined heptane extracts was added the aqueous NaCl solution. The batch was agitated for 5 to 10 minutes, and the layers were separated. The first aqueous sodium bicarbonate solution was added. The batch was agitated for 10 minutes and the layers were separated. DMF (175 mL) was added. Heptane was distilled off under a vacuum of approximately 130 mmHg and a jacket temperature of 50°C-55°C. The imidazole/DMF solution was added followed by TMSCl at a rate that internal temperature did not exceed 30°C. The reaction was held at 20°C-25°C for no less than 20 hours. Heptane (500 mL) was added followed by water (500 mL) at a rate that internal temperature did not exceed 25°C. The batch was agitated for 10 minutes and the layers were separated. The second aqueous sodium bicarbonate solution was added. The batch was agitated for 10 minutes and the layers were separated. Water (300 mL) was added. The batch was agitated for 10 minutes and the layers were separated. Heptane (355.8 g, -515 mL) was distilled off at a vacuum of approximately 130 mmHg and a jacket temperature of 50°C-55°C. 189.4 g of a solution of the product in heptane was obtained which was 88.6 wt.% O-trimethyl silyl protected (R)-5-(5-fluoro-2-methylphenyl)-3-hydroxy-5-methyl-3-trifluoromethylhexanoic acid methyl ester and 10.6 wt.% heptane by assay. Assay yield: 167.81 g, 96.8% over two steps.

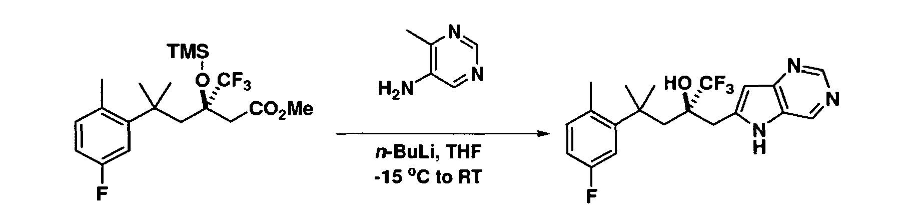

### Stage 8:

225.0 g (2.06 mol) of 4-methyl-5-aminopyrimidine was dissolved in 10.0 L of anhydrous THF in a dry flask under nitrogen blanket. The solution was cooled to -20°C. 2.0 L (1.39 kg, 5.00 mol) of 2.5 M *n-*BuLi in hexane was added in 20 minutes while the temperature was kept below -5°C. The mixture was agitated at below -15°C for 30 minutes and then warmed up to normal room temperature (RT) and stirred for 3 hours. 250.0 g (0.61 mol) of the above methyl ester from stage 7, in 250 mL of anhydrous THF, was added over 25 minutes while the temperature was kept below 35°C. The light yellow slurry turned to a dark solution. The solution was stirred for 20 minutes. A reaction sample was quenched with MeOH and analyzed by HPLC to make certain that there is no starting methyl ester left. The solution was then cooled back to below 15°C. 2.0 L of MeOH was added over 10 minutes while the temperature was kept below 25°C followed by 1.0 L of water added in one portion and the mixture was allowed to warm up to room temperature and stir for 15 hours. A reaction sample was analyzed by HPLC to make certain all related intermediates have been converted. The mixture was cooled to below 20°C. 1.2 L of 6 N hydrochloric acid was added over 35 minutes while the temperature was kept below 25°C. The solution was agitated for 1 hour. The reaction was monitored by HPLC to make certain that all related intermediates had been converted. The solution was cooled to below 15°C. 6 N of NaOH was added to adjust the solution pH to 7-8 (about 370 mL was needed). Most solvents (14.5 L) were removed under vacuum (140 mmHg) and 4.0 L of EtOAc and 2.0 L of water were added. Layers were separated and the organic layer was washed with three 2.0 L portions of water and 1.0 L of brine. The solvent was removed under vacuum from the organic layer to yield 450.0 g of a thick dark brown oil as the crude product. The residue was chased with 500 mL of *n*-propanol to get 393.2 g of a thick slurry. 500 mL of *n*-propanol was added and the mixture was heated up to 60°C to dissolve the solid. The solution was cooled down with agitation and the slurry was filtered after 16 hours at room temperature and the solid was washed with the filtrate and two 100 mL portions of *n-*propanol and two 200 mL portions of hexane and air dried to yield the title compound (*R*)-1,1,1-trifluoro-4-(5-fluoro-2-methylphenyl)-4-methyl-2-(5*H*-pyrrolo[3,2-*d*]pyrimidin-6-ylmethyl)pentan-2-ol *n*-propanol solvate as a light yellow solid, 140.2 g, 44% yield.

## Claims

1. A process for stereoselective synthesis of a compound of Formula (**I**) 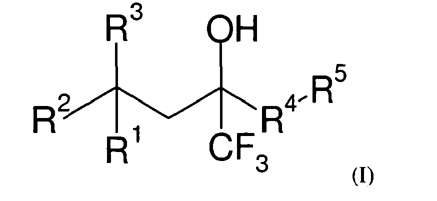 wherein:
R¹ is an aryl or heteroaryl group, each optionally substituted with one to three substituent groups,
wherein each substituent group of R¹ is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, C₂-C₅ alkynyloxy, aryloxy, C₁-C₅ alkanoyloxy, C₁-C₅ alkanoyl, aroyl, halogen, trifluoromethyl, trifluoromethoxy, or C₁-C₅ alkylthio,
wherein each substituent group of R¹ is optionally independently substituted with one to three substituent groups selected from methyl, methoxy, fluoro, chloro, or alkoxy;
R² and R³ are each independently hydrogen or C₁-C₅ alkyl, or R² and R³ together with the carbon atom they are commonly attached to form a C₃-C₈ spiro cycloalkyl ring;
R⁴ is C₁-C₅ alkyl, C₂-C₅ alkenyl, or C₂-C₅ alkynyl, each optionally substituted with one to three substituent groups,
wherein each substituent group of R⁴ is independently C₁-C₃ alkyl, hydroxy, halogen, amino, or oxo; and
R⁵ is the moiety 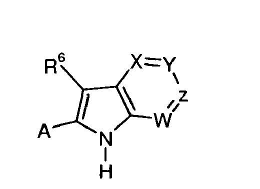
wherein A is the point of attachment to R⁴,
W, X, Y, or Z is N or CH and at least two of W, X, Y, or Z is N, and
R⁶ is H, alkyl, or aryl, and
R⁵ is optionally substituted with one to three substituent groups, wherein each substituent group of R⁵ is independently C₁-C₅ alkyl, C₂-C₅ alkenyl, C₂-C₅ alkynyl, C₃-C₈ cycloalkyl, heterocyclyl, aryl, heteroaryl, C₁-C₅ alkoxy, C₂-C₅ alkenyloxy, *C₂*-C₅ alkynyloxy, aryloxy, acyl, C₁-C₅ alkoxycarbonyl, C₁-C₅ alkanoyloxy, aminocarbonyl, alkylaminocarbonyl, dialkylaminocarbonyl, aminocarbonyloxy, C₁-C₅ alkylaminocarbonyloxy, C₁-C₅ dialkylaminocarbonyloxy, C₁-C₅ alkanoylamino, C₁-C₅ alkoxycarbonylamino, C₁-C₅ alkylsulfonylamino, aminosulfonyl, C₁-C₅ alkylaminosulfonyl, C₁-C₅ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by C₁-C₅ alkyl; or ureido wherein either nitrogen atom is optionally independently substituted with C₁-C₅ alkyl; or C₁-C₅ alkylthio wherein the sulfur atom is optionally oxidized to a sulfoxide or sulfone,
wherein each substituent group of R⁵ is optionally independently substituted with one to three substituent groups selected from C₁-C₃ alkyl, C₁-C₃ alkoxy, halogen, hydroxy, oxo, cyano, amino, or trifluoromethyl,
the process comprising:
(a) reacting a starting material of formula A with an unsaturated ester of formula B in the presence of a salt, without a solvent or with a suitable solvent, at a suitable temperature to provide an ester of formula C 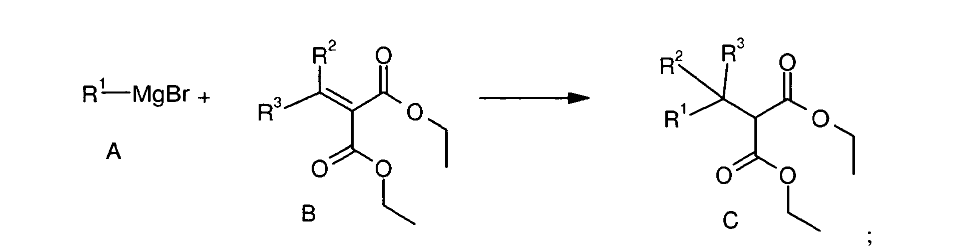
(b) hydrolyzing the ester of formula C using a suitable acid in water, with or without an organic solvent, at a suitable temperature to provide an acid of formula D 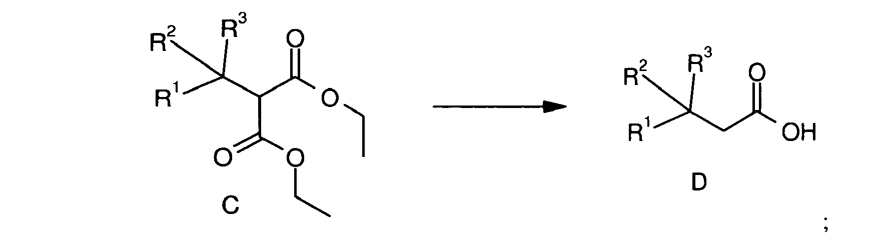
(c) reacting the acid of formula D with suitable trifluoroacetate in the presence of a suitable base in a suitable solvent at a suitable temperature to provide a trifluoromethyl ketone of formula E 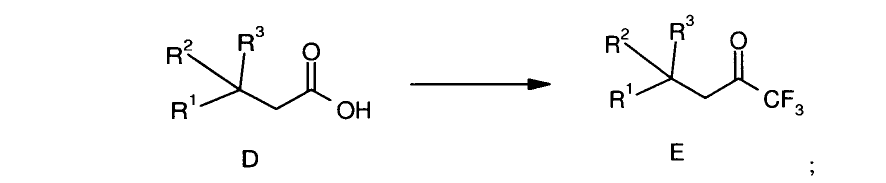
(d) reacting the trifluoromethyl ketone of formula E with an acetate in the presence of a suitable base in a suitable solvent at a suitable temperature to prepare an acid of formula F 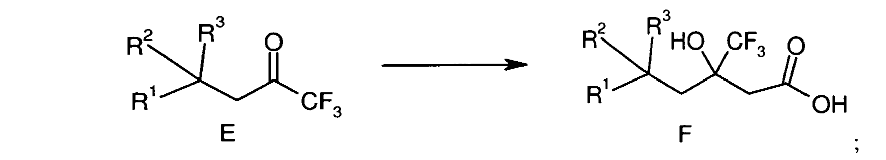
(e) reacting the acid of formula F with a suitable resolving base to provide a pure diastereomer followed by reacting the pure diastereomer with a suitable base in a suitable solvent at a suitable temperature to provide a pure enantiomer of formula G, or reacting a pure diastereomer of the acid of formula F with a suitable acid in a suitable
solvent at a suitable temperature to obtain a pure enantiomer of formula G 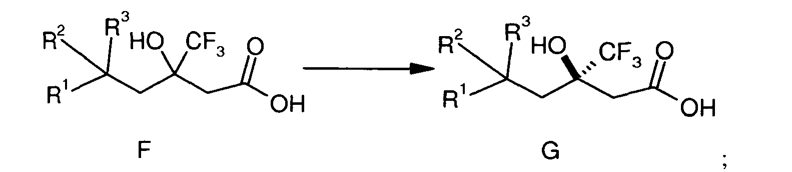
(f) reacting the acid of formula G with a suitable alcohol, R'-OH, where R' is an alkyl group, followed by protection of the tertiary alcohol with a protecting group agent PG-Y, where Y is a leaving group, at a suitable temperature to obtain the ester of formula H 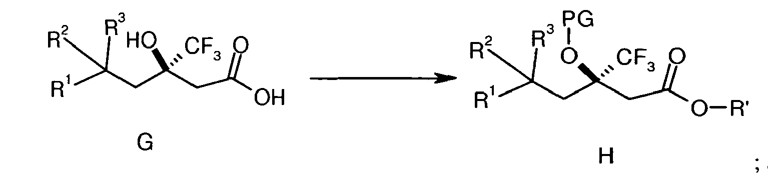 and
(g) reacting the ester of formula H with a compound of formula J, wherein W, X, Y, or Z is N or CH, at least two of W, X, Y, or Z is N, and R⁶ is H, alkyl, or aryl, in the presence of a suitable base in a suitable solvent at a suitable temperature to obtain a compound of Formula (I)

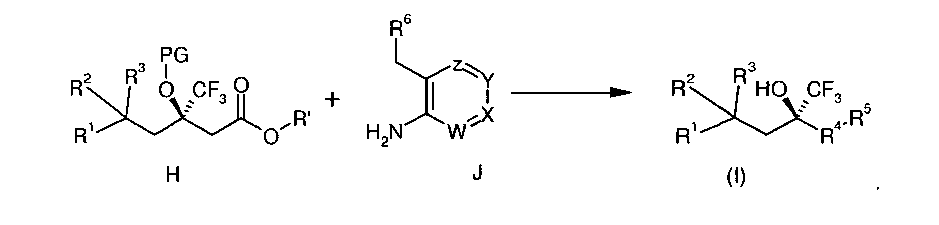

2. The process according to claim 1, wherein the suitable solvent of step (a) is THF, diethyl ether, dimethyl ether, dipropyl ether, diisopropyl ether, dibutyl ether, DME, MTBE, or a mixture thereof.

3. The process according to claim 1, wherein the suitable salt of step (a) is copper (I) chloride, copper (I) bromide, or copper (I) triflate, preferably copper (I) chloride.

4. The process according to claim 3, wherein the suitable salt of step (a) is copper (I) chloride.

5. The process according to claim 1, wherein the suitable solvent of step (b) is water, MeOH, EtOH, dimethyl ether, diethyl ether, dipropyl ether, diisopropyl ether, dibutyl ether, THF, DME, MTBE, or a mixture thereof.

6. The process according to claim 1, wherein the suitable acid of step (b) is acetic acid, sulfuric acid, hydrochloric acid, or a mixture thereof.

7. The process according to claim 1, wherein the suitable solvent of step (c) is toluene, xylene, heptane, dimethyl ether, diethyl ether, dipropyl ether, diisopropyl ether, dibutyl ether, THF, DME, MTBE, or a mixture thereof.

8. The process according to claim 1, wherein the suitable base of step (c) is methyl lithium, *n*-BuLi, *sec*-BuLi, *tert*-BuLi, LDA, LiHMDS, NaHMDS, or KHMDS.

9. The process according to claim 1, wherein the suitable solvent of step (d) is THF, DME, diethyl ether, dipropyl ether, diisopropyl ether, dibutyl ether, MTBE, toluene, xylene, or DMF.

10. The process according to claim 1, wherein the suitable base of step (d) is LiHMDS, NaHMDS, KHMDS, LDA, LiH, NaH, KH, or NaNH₂.

11. The process according to claim 1, wherein the suitable acetate reagent of step (d) is methyl acetate, ethyl acetate, propyl acetate, or butyl acetate.

12. The process according to claim 1, wherein the suitable resolving base of step (e) is (+ or -) *cis*-1-amino-2-indanol, quinine, quinidine, (+ or -) ephedrine, (+ or -) deoxyephedrine, (+ or -) methylbenzylamine, (+ or -) (1-naphthyl)ethylamine, or (+ or -) (2-naphthyl)ethylamine.

13. The process according to claim 1, wherein the suitable base of step (e) is potassium hydroxide, lithium hydroxide, sodium bicarbonate, sodium carbonate, potassium bicarbonate, potassium carbonate, or lithium carbonate.

14. The process according to claim 1, the suitable solvent of step (e) is dichloromethane, diethyl ether, ethyl acetate, isopropyl acetate, *n*-butyl acetate, heptane, hexane, toluene, xylene, MTBE, or a mixture thereof

15. The process according to claim 1, wherein the esterification of step (f) is carried out by treatment of the free acid with a lower alkyl alcohol in the presence of an acid catalyst selected from sulfuric acid, hydrochloric acid, acetic acid, *p*-toluenesulfonic acid, and acetic acid.

16. The process according to claim 1, wherein the suitable solvent of step (f) is dichloromethane, DMF, THF, diethyl ether, dipropyl ether, diisopropyl ether, dibutyl ether, DME, ethyl acetate, isopropyl acetate, *n*-butyl acetate, heptane, hexane, toluene, xylene, MTBE, or a mixture thereof.

17. The process according to claim 1, wherein the suitable solvent of step (g) is THF, DME, diethyl ether, dipropyl ether, diisopropyl ether, dibutyl ether, MTBE, toluene, benzene, xylene, hexane, pentane, heptane, methylene chloride, or a mixture thereof.

18. The process according to claim 1, wherein the suitable base of step (g) is *n*-BuLi, *sec-*BuLi, *tert*-BuLi, or LDA, optionally including additives such as *N,N,N',N'-*tetramethylethylenediamine (TMEDA), β-dialkylaminoalcohols, sparteine, or polyethers.

19. The process according to claim 1, wherein the compound of formula J is 4-methyl-5-aminopyrimidine, 4-amino-5-methylpyrimidine, 5-amino-6-methylpyrimidine, or 5-methyl-6-aminopyrimidine, each optionally substituted on the ring or methyl group with a substituent compatible with alkyl lithium.

## Patentansprüche

1. Verfahren zur stereoselektiven Synthese einer Verbindung der Formel (I) 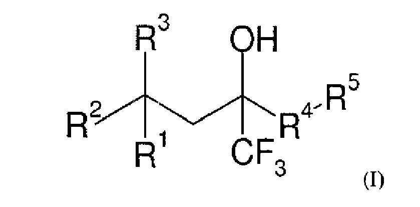 wobei
R¹ eine Aryl- oder Heteroarylgruppe bedeutet, die optional jeweils mit 1 bis 3 Substituentengruppen substituiert ist,
wobei jede Substituentengruppe von R¹ unabhängig voneinander C₁-C₅-Alkyl, C₂-C₅-Alkenyl, C₂-C₅-Alkinyl, C₃-C₈-Cycloalkyl, Heterocyclyl, Aryl, Heteroaryl, C₁-C₅-Alkoxy, C₂-C₅-Alkenyloxy, C₂-C₅-Alkinyloxy, Aryloxy, C₁-C₅-Alkanoyloxy, C₁-C₅-Alkanoyl, Aroyl, Halogen, Trifluormethyl, Trifluormethoxy oder C₁-C₅-Alkylthio bedeutet,
wobei jede Substituentengruppe von R¹ optional unabhängig voneinander mit 1 bis 3 Substituentengruppen substituiert ist, die aus Methyl, Methoxy, Fluor, Chlor oder Alkoxy ausgewählt sind;
R² und R³ jeweils unabhängig voneinander Wasserstoff oder C₁-C₅-Alkyl bedeuten oder R² und R³ zusammen mit dem Kohlenstoffatom, an das sie gemeinsam gebunden sind, einen C₃-C₈-Spirocycloalkylring bilden;
R⁴ C₁-C₅-Alkyl, C₂-C₅-Alkenyl oder C₂-C₅-Alkinyl bedeutet, die jeweils optional mit 1 bis 3 Substituentengruppen substituiert sind,
wobei jede Substituentengruppe von R⁴ unabhängig voneinander C₁-C₃-Alkyl, Hydroxy, Halogen, Amino oder Oxo bedeutet; und
R⁵ den folgenden Rest bedeutet 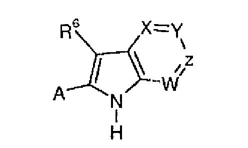
wobei A die Bindungsstelle an R⁴ bedeutet,
W, X, Y oder Z die Bedeutung N oder CH haben und mindestens zwei der Reste W, X, Y oder Z die Bedeutung N haben und
R⁶ H, Alkyl oder Aryl bedeutet; und
R⁵ optional substituiert ist mit: 1 bis 3 Substituentengruppen, wobei jede Substituentengruppe von R⁵ unabhängig voneinander C₁-C₅-Alkyl, C₂-C₅-Alkenyl, C₂-C₅-Alkinyl, C₃-C₈-Cycloalkyl, Heterocyclyl, Aryl, Heteroaryl, C₁-C₅-Alkoxy, C₂-C₅-Alkenyloxy, C₂-C₅-Alkinyloxy, Aryloxy, Acyl, C₁-C₅-Alkoxycarbonyl, C₁-C₅-Alkanoyloxy, Aminocarbonyl, Alkylaminocarbonyl, Dialkylaminocarbonyl, Aminocarbonyloxy, C₁-C₅-Alkylaminocarbonyloxy, C₁-C₅-Dialkylaminocarbonyloxy, C₁-C₅-Alkanoylamino, C₁-C₅-Alkoxycarbonylamino, C₁-C₅-Alkylsulfonylamino, Aminosulfonyl, C₁-C₅-Alkylaminosulfonyl, C₁-C₅-Dialkylaminosulfonyl, Halogen, Hydroxy, Carboxy, Cyano, Trifluormethyl, Trifluormethoxy, Trifluormethylthio, Nitro oder Amino bedeutet, wobei das Stickstoffatom optional unabhängig voneinander durch C₁-C₅- mono- oder disubstituiert ist; oder Ureido, wobei jedes Stickstoffatom optional unabhängig voneinander mit C₁-C₅-Alkyl substituiert ist; oder C₁-C₅-Alkylthio, wobei das Schwefelatom optional zu einem Sulfoxid oder Sulfon oxidiert ist,
wobei jede Substituentengruppe von R⁵ optional unabhängig voneinander mit 1 bis 3 Substituentengruppen substituiert ist, die aus C₁-C₃-Alkyl, C₁-C₃-Alkoxy, Halogen, Hydroxy, Oxo, Cyano, Amino oder Trifluormethyl ausgewählt sind;
wobei das Verfahren Folgendes umfasst:
(a) Umsetzen eines Ausgangsmaterials der Formel A mit einem ungesättigten Ester der Formel B in Gegenwart eines Salzes ohne ein Lösungsmittel oder mit einem geeigneten Lösungsmittel bei einer geeigneten Temperatur, um einen Ester der Formel C zu bilden 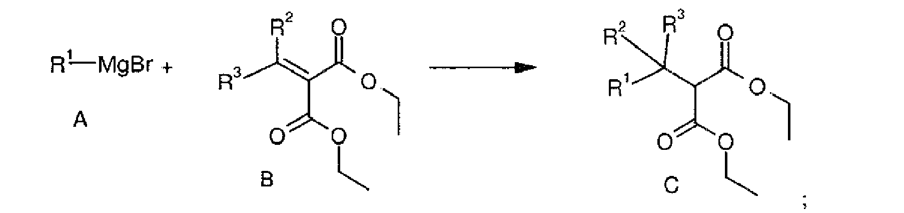
(b) Hydrolysieren des Esters der Formel C unter Verwendung einer geeigneten Säure in Wasser mit oder ohne ein organisches Lösungsmittel bei einer geeigneten Temperatur, um eine Säure der Formel D zu bilden 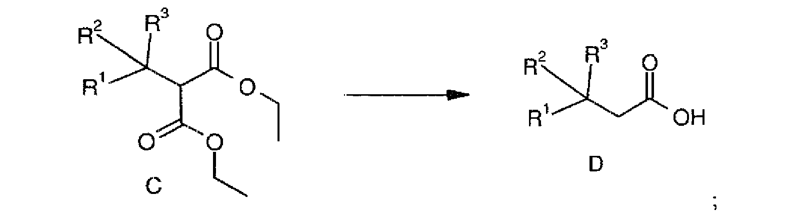
(c) Umsetzen der Säure der Formel D mit einem geeigneten Trifluoracetat in Gegenwart einer geeigneten Base in einem geeigneten Lösungsmittel bei einer geeigneten Temperatur, um ein Trifluormethylketon der Formel E zu bilden 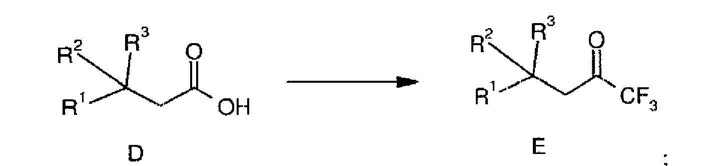
(d) Umsetzen des Trifluormethylketons der Formel E mit einem Acetat in Gegenwart einer geeigneten Base in einem geeigneten Lösungsmittel bei einer geeigneten Temperatur zur Herstellung einer Säure der Formel F 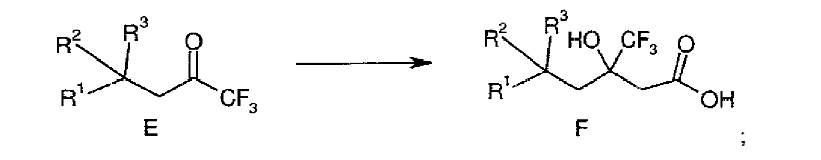
(e) Umsetzen der Säure der Formel F mit einer geeigneten Base zur optischen Auftrennung zur Bereitstellung eines reinen Diastereomeren, gefolgt von der Umsetzung des reinen Diastereomeren mit einer geeigneten Base in einem geeigneten Lösungsmittel bei einer geeigneten Temperatur, um ein reines Enantiomeres der Formel G zu bilden, oder Umsetzen eines reinen Diastereomeren der Säure der Formel F mit einer geeigneten Säure in einem geeigneten Lösungsmittel bei einer geeigneten Temperatur, um ein reines Enantiomeres der Formel G zu bilden 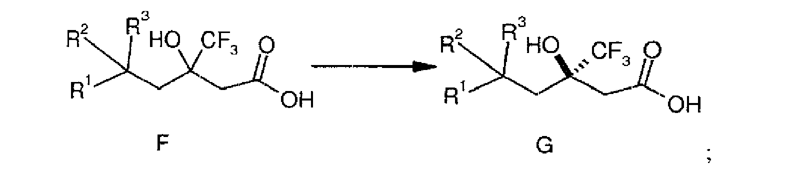
(f) Umsetzen der Säure der Formel G mit einem geeigneten Alkohol R'-OH, wobei R' eine Alkylgruppe bedeutet, gefolgt vom Schützen des tertiären Alkohols mit einem Schutzgruppenmittel PG-Y, wobei Y eine austretende Gruppe bedeutet, bei einer geeigneten Temperatur, um den Ester der Formel H zu bilden 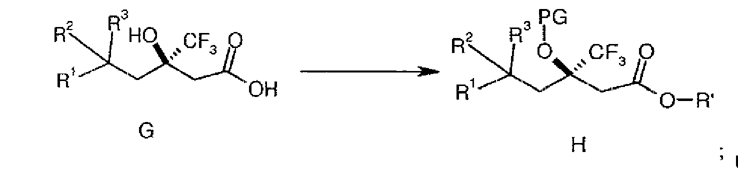 und
(g) Umsetzen des Esters der Formel H mit einer Verbindung der Formel J, wobei W, X, Y oder Z die Bedeutung N oder CH haben, wobei mindestens zwei der Reste W, X, Y oder Z die Bedeutung N haben, und R⁶ H, Alkyl oder Aryl bedeutet, in Gegenwart einer geeigneten Base in einem geeigneten Lösungsmittel bei einer geeigneten Temperatur, um eine Verbindung der Formel (I) zu bilden

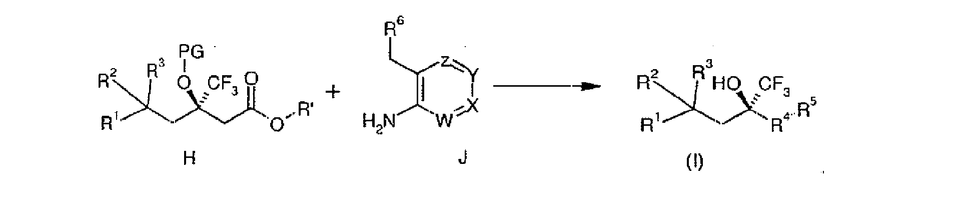

2. Verfahren nach Anspruch 1, wobei es sich beim geeigneten Lösungsmittel von Stufe (a) um THF, Diethylether, Dimethylether, Dipropylether, Diisopropylether, Dibutylether, DME, MTBE oder ein Gemisch davon handelt.

3. Verfahren nach Anspruch 1, wobei es sich beim geeigneten Salz von Stufe (a) um Kupfer(I)-chlorid, Kupfer(I)-bromid oder Kupfer(I)-triflat und vorzugsweise Kupfer(I)-chlorid handelt.

4. Verfahren nach Anspruch 3, wobei es sich beim geeigneten Salz von Stufe (a) um Kupfer(I)-chlorid handelt.

5. Verfahren nach Anspruch 1, wobei es sich beim geeigneten Lösungsmittel von Stufe (b) um Wasser, MeOH, EtOH, Dimethylether, Diethylether, Dipropylether, Diisopropylether, Dibutylether, THF, DME, MTBE oder ein Gemisch davon handelt.

6. Verfahren nach Anspruch 1, wobei es sich bei der geeigneten Säure von Stufe (b) um Essigsäure, Schwefelsäure, Chlorwasserstoffsäure oder ein Gemisch davon handelt.

7. Verfahren nach Anspruch 1, wobei es sich beim geeigneten Lösungsmittel von Stufe (c) um Toluol, Xylol, Heptan, Dimethylether, Diethylether, Dipropylether, Diisopropylether, Dibutylether, THF, DME, MTBE oder ein Gemisch davon handelt.

8. Verfahren nach Anspruch 1, wobei es sich bei der geeigneten Base von Stufe (c) um Methyllithium, n-BuLi, sec.-BuLi, tert.-BuLi, LDA, LiHMDS, NaHMDS oder KHMDS handelt.

9. Verfahren nach Anspruch 1, wobei es sich beim geeigneten Lösungsmittel von Stufe (d) um THF, DME, Diethylether, Dipropylether, Diisopropylether, Dibutylether, MTBE, Toluol, Xylol oder DMF handelt.

10. Verfahren nach Anspruch 1, wobei es sich bei der geeigneten Base von Stufe (d) um LiHMDS, NaHMDS, KHMDS, LDA, LiH, NaH, KH oder NaNH₂ handelt.

11. Verfahren nach Anspruch 1, wobei es sich beim geeigneten Acetatreagenz von Stufe (d) um Methylacetat, Ethylacetat, Propylacetat oder Butylacetat handelt.

12. Verfahren nach Anspruch 1, wobei es sich bei der geeigneten Base zur optischen Auftrennung von Stufe (e) um (+ oder -)-cis-1-Amino-2-indanol, Chinin, Chinidin, (+ oder -)-Ephedrin, (+ oder -)-Deoxyephedrin, (+ oder -)-Methylbenzylamin, (+ oder -)-(1-Naphthyl)-ethylamin oder (+ oder -)-(2-Naphthyl)-ethylamin handelt.

13. Verfahren nach Anspruch 1, wobei es sich bei der geeigneten Base von Stufe (e) um Kaliumhydroxid, Lithiumhydroxid, Natriumbicarbonat, Natriumcarbonat, Kaliumbicarbonat, Kaliumcarbonat oder Lithiumcarbonat handelt.

14. Verfahren nach Anspruch 1, wobei es sich beim geeigneten Lösungsmittel von Stufe (e) um Dichlormethan, Diethylether, Ethylacetat, Isopropylacetat, n-Butylacetat, Heptan, Hexan, Toluol, Xylol, MTBE oder ein Gemisch davon handelt.

15. Verfahren nach Anspruch 1, wobei die Veresterung von Stufe (f) durch Behandlung der freien Säure mit einem niederen Alkylalkohol in Gegenwart eines Säurekatalysators, der aus Schwefelsäure, Chlorwasserstoffsäure, Essigsäure, p-Toluolsulfonsäure und Essigsäure ausgewählt ist, durchgeführt wird.

16. Verfahren nach Anspruch 1, wobei es sich beim geeigneten Lösungsmittel von Stufe (f) um Dichlormethan, DMF, THF, Diethylether, Dipropylether, Diisopropylether, Dibutylether, DME, Ethylacetat, Isopropylacetat, n-Butylacetat, Heptan, Hexan, Toluol, Xylol, MTBE oder ein Gemisch davon handelt.

17. Verfahren nach Anspruch 1, wobei es sich beim geeigneten Lösungsmittel von Stufe (g) um THF, DME, Diethylether, Dipropylether, Diisopropylether, Dibutylether, MTBE, Toluol, Benzol, Xylol, Hexan, Pentan, Heptan, Methylenchlorid oder ein Gemisch davon handelt.

18. Verfahren nach Anspruch 1, wobei es sich bei der geeigneten Base von Stufe (g) um n-BuLi, sec.-BuLi, tert.-BuLi oder LDA handelt, die gegebenenfalls Additive, wie N,N,N',N'-Tetramethylethylendiamin (TMEDA), β-Dialkylaminoalkohole, Spartein oder Polyether, enthalten.

19. Verfahren nach Anspruch 1, wobei es sich bei der Verbindung der Formel um 4-Methyl-5-aminopyrimidin, 4-Amino-5-methylpyrimidin, 5-Amino-6-methylpyrimidin oder 5-Methyl-6-aminopyrimidin handelt, die jeweils optional am Ring oder an der Methylgruppe mit einem Substituenten, der mit Alkyllithium verträglich ist, substituiert sind.

## Revendications

1. Procédé de synthèse stéréosélective d'un composé de formule (I) 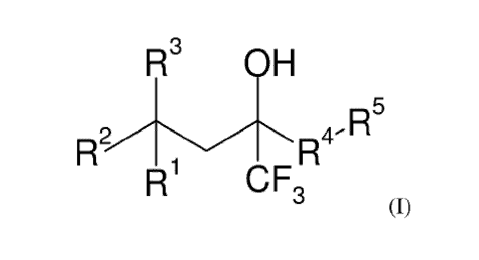 dans laquelle :
R¹ est un groupe aryle ou hétéroaryle, chacun facultativement substitué par un à trois groupes substituants,
où chaque groupe substituant de R¹ est indépendamment un groupe alkyle en C₁ à C₅, alcényle en C₂ à C₅, alcynyle en C₂ à C₅, cycloalkyle en C₃ à C₈, hétérocyclyle, aryle, hétéroaryle, alcoxy en C₁ à C₅, alcényloxy en C₂ à C₅, alcynyloxy en C₂ à C₅, aryloxy, alcanoyloxy en C₁ à C₅, alcanoyle en C₁ à C₅, aroyle, un atome d'halogène, un groupe trifluorométhyle, trifluorométhoxy ou alkylthio en C₁ à C₅,
où chaque groupe substituant de R¹ est facultativement indépendamment substitué par un à trois groupes substituants choisis parmi un groupe méthyle, méthoxy, fluoro, chloro ou alcoxy ;
R² et R³ sont chacun indépendamment un atome d'hydrogène ou un groupe alkyle en C₁ à C₅, ou bien R² et R³ conjointement avec l'atome de carbone auquel ils sont communément attachés forment un cycle spirocycloalkyle en C₃ à C₈ ;
R⁴ est un groupe alkyle en C₁ à C₅, alcényle en C₂ à C₅ ou alcynyle en C₂ à C₅, chacun facultativement substitué par un à trois groupes substituants,
où chaque groupe substituant de R⁴ est indépendamment un groupe alkyle en C₁ à C₃, hydroxy, un atome d'halogène, un groupe amino ou oxo ; et
R⁵ est le fragment 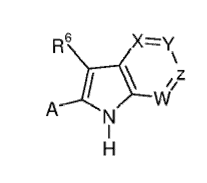
dans lequel A est le point d'attache à R⁴, W, X, Y ou Z est N ou CH et au moins deux de W, X, Y ou Z sont N, et
R⁶ est H, un groupe alkyle, ou aryle, et
R⁵ est facultativement substitué par un à trois groupes substituants, où chaque groupe substituant de R⁵ est indépendamment un groupe alkyle en C₁ à C₅, alcényle en C₂ à C₅, alcynyle en C₂ à C₅, cycloalkyle en C₃ à C₈, hétérocyclyle, aryle, hétéroaryle, alcoxy en C₁ à C₅, alcényloxy en C₂ à C₅, alcynyloxy en C₂ à C₅, aryloxy, acyle, alcoxycarbonyle en C₁ à C₅, alcanoyloxy en C₁ à C₅, aminocarbonyle, alkylaminocarbonyle, dialkylaminocarbonyle, aminocarbonyloxy, alkylaminocarbonyloxy en C₁ à C₅, dialkylaminocarbonyloxy en C₁ à C₅, alcanoylamino en C₁ à C₅, alcoxycarbonylamino en C₁ à C₅, alkylsulfonylamino en C₁ à C₅, aminosulfonyle, alkylaminosulfonyle en C₁ à C₅, dialkylaminosulfonyle en C₁ à C₅, un atome d'halogène, un groupe hydroxy, carboxy, cyano, trifluorométhyle, trifluorométhoxy, trifluorométhylthio, nitro ou amino où l'atome d'azote est facultativement indépendamment mono- ou di-substitué par un groupe alkyle en C₁ à C₅ ; 1 ou uréido dans lequel l'un ou l'autre des atomes d'azote est facultativement indépendamment substitué par un groupe alkyle en C₁ à C₅ ; ou alkylthio en C₁ à C₅ dans lequel l'atome de soufre est facultativement oxydé en sulfoxyde ou sulfone,
où chaque groupe substituant de R⁵ est facultativement indépendamment substitué par un à trois groupes substituants choisis parmi un groupe alkyle en C₁ à C₃, alcoxy en C₁ à C₃, un atome d'halogène, un groupe hydroxy, oxo, cyano, amino ou trifluorométhyle,
le procédé comprenant les étapes consistant à :
(a) faire réagir une matière de départ de formule A avec un ester insaturé de formule B en présence d'un sel, sans solvant ou avec un solvant approprié, à une température appropriée pour former un ester de formule C 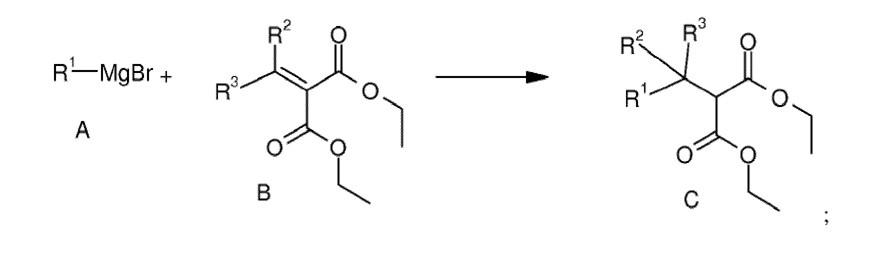
(b) hydrolyser l'ester de formule C à l'aide d'un acide approprié dans l'eau, avec ou sans solvant organique, à une température appropriée pour former un acide de formule D 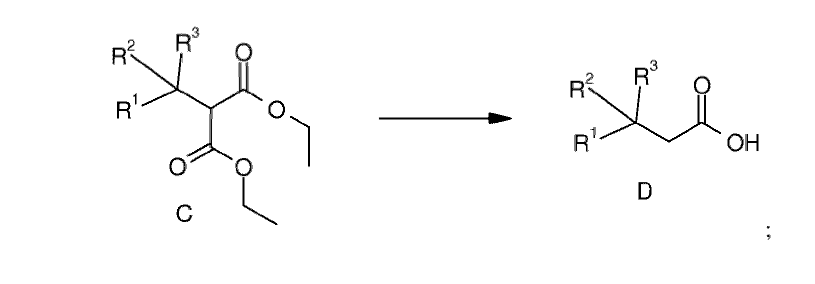
(c) faire réagir l'acide de formule D avec un trifluoroacétate approprié en présence d'une base appropriée dans un solvant approprié à une température appropriée pour former une trifluorométhylcétone de formule E 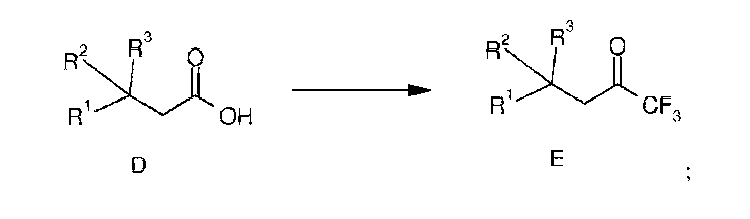
(d) faire réagir la trifluorométhylcétone de formule E avec un acétate en présence d'une base appropriée dans un solvant approprié à une température appropriée pour préparer un acide de formule F 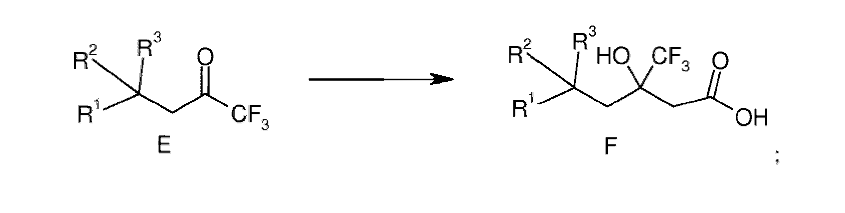
(e) faire réagir l'acide de formule F avec une base de résolution appropriée pour former un diastéréoisomère pur puis faire réagir le diastéréoisomère pur avec une base appropriée dans un solvant approprié à une température appropriée pour former un énantiomère pur de formule G, ou faire réagir un diastéréoisomère pur de l'acide de formule F avec un acide approprié dans un solvant approprié à une température appropriée pour obtenir un énantiomère pur de formule G 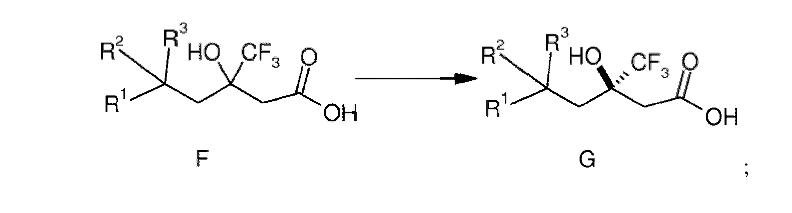
(f) faire réagir l'acide de formule G avec un alcool approprié, R'-OH, où R' est un groupe alkyle, puis protéger l'alcool tertiaire avec un groupe protecteur PG-Y, où Y est un groupe partant, à une température appropriée pour obtenir l'ester de formule H 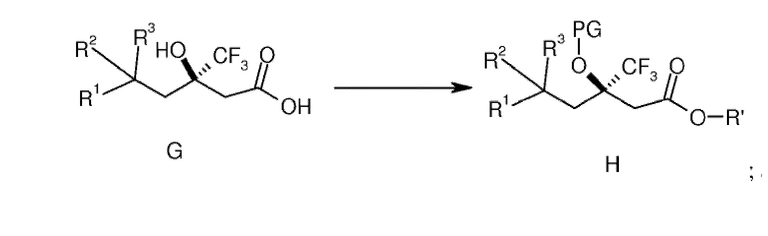 et
(g) faire réagir l'ester de formule H avec un composé de formule J, dans lequel W, X, Y ou Z est N ou CH, au moins deux de W, X, Y ou Z sont N, et R⁶ est H, un groupe alkyle ou aryle, en présence d'une base appropriée dans un solvant approprié à une température appropriée pour obtenir un composé de formule (I)

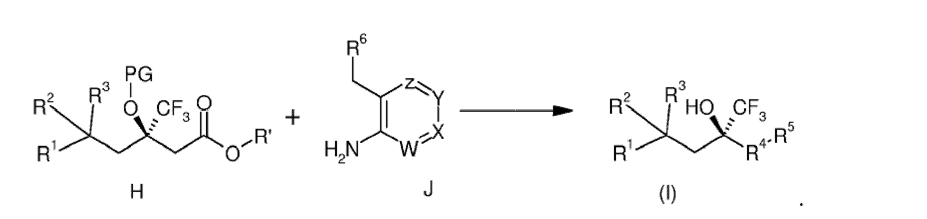

2. Procédé selon la revendication 1, dans lequel le solvant approprié de l'étape (a) est le THF, le diéthyléther, le diméthyléther, le dipropyléther, le diisopropyléther, le dibutyléther, le DME, le MTBE, ou un de leurs mélanges.

3. Procédé selon la revendication 1, dans lequel le sel approprié de l'étape (a) est le chlorure de cuivre (I), le bromure de cuivre (I) ou le triflate de cuivre (I), de préférence le chlorure de cuivre (I).

4. Procédé selon la revendication 3, dans lequel le sel approprié de l'étape (a) est le chlorure de cuivre (I).

5. Procédé selon la revendication 1, dans lequel le solvant approprié de l'étape (b) est l'eau, le MeOH, l'EtOH, le diméthyléther, le diéthyléther, le dipropyléther, le diisopropyléther, le dibutyléther, le THF, le DME, le MTBE, ou un de leurs mélanges.

6. Procédé selon la revendication 1, dans lequel l'acide approprié de l'étape (b) est l'acide acétique, l'acide sulfurique, l'acide chlorhydrique, ou l'un de leurs mélanges.

7. Procédé selon la revendication 1, dans lequel le solvant approprié de l'étape (c) est le toluène, le xylène, l'heptane, le diméthyléther, le diéthyléther, le dipropyléther, le diisopropyléther, le dibutyléther, le THF, le DME, le MTBE, ou un de leurs mélanges.

8. Procédé selon la revendication 1, dans lequel la base appropriée de l'étape (c) est le méthyl lithium, le *n-*BuLi, le sec-BuLi, le tert-BuLi, le LDA, le LiHMDS, le NaHMDS ou le KHMDS.

9. Procédé selon la revendication 1, dans lequel le solvant approprié de l'étape (d) est le THF, le DME, le diéthyléther, le dipropyléther, le diisopropyléther, le dibutyléther, le MTBE, le toluène, le xylène ou le DMF.

10. Procédé selon la revendication 1, dans lequel la base appropriée de l'étape (d) est le LiHMDS, le NaHMDS, le KHMDS, le LDA, le LiH, le NaH, le KH ou le NaNH₂.

11. Procédé selon la revendication 1, dans lequel le réactif acétate approprié de l'étape (d) est l'acétate de méthyle, l'acétate d'éthyle, l'acétate de propyle ou l'acétate de butyle.

12. Procédé selon la revendication 1, dans lequel la base de résolution appropriée de l'étape (e) est le (+ ou -)*cis*-1-amino-2-indanol, la quinine, la quinidine, la (+ ou -)éphédrine, la (+ ou -)désoxyéphédrine, la (+ ou -)méthylbenzylamine, la (+ ou -) (1-naphtyl)éthylamine ou la (+ ou -)(2-naphtyl)éthylamine.

13. Procédé selon la revendication 1, dans lequel la base appropriée de l'étape (e) est l'hydroxyde de potassium, l'hydroxyde de lithium, le bicarbonate de sodium, le carbonate de sodium, le bicarbonate de potassium, le carbonate de potassium ou le carbonate de lithium.

14. Procédé selon la revendication 1, dans lequel le solvant approprié de l'étape (e) est le dichlorométhane, le diéthyléther, l'acétate d'éthyle, l'acétate d' isopropyle , l'acétate de *n-*butyle, l'heptane, l'hexane, le toluène, le xylène, le MTBE, ou un de leurs mélanges.

15. Procédé selon la revendication 1, dans lequel l'estérification de l'étape (f) est réalisée par traitement de l'acide libre avec un alcool d'alkyle inférieur en présence d'un catalyseur acide choisi parmi l'acide sulfurique, l'acide chlorhydrique, l'acide acétique, l'acide p-toluènesulfonique et l'acide acétique.

16. Procédé selon la revendication 1, dans lequel le solvant approprié de l'étape (f) est le dichlorométhane, le DMF, le THF, le diéthyléther, le dipropyléther, le diisopropyléther, le dibutyléther, le DME, l'acétate d'éthyle, l'acétate d'isopropyle, l'acétate de *n-*butyle, l'heptane, l'hexane, le toluène, le xylène, le MTBE, ou un de leurs mélanges.

17. Procédé selon la revendication 1, dans lequel le solvant approprié de l'étape (g) est le THF, le DME, le diéthyléther, le dipropyléther, le diisopropyléther, le dibutyléther, le MTBE, le toluène, le benzène, le xylène, l'hexane, le pentane, l'heptane, le chlorure de méthylène, ou un de leurs mélanges.

18. Procédé selon la revendication 1, dans lequel la base appropriée de l'étape (g) est le *n-*BuLi, le sec-BuLi, le tert-BuLi ou le LDA, comprenant facultativement des additifs tels que la N,N,N',N'-tétraméthyléthylènediamine (TMEDA), les β-dialkylaminoalcools, la spartéine ou les poly(éthers).

19. Procédé selon la revendication 1, dans lequel le composé de formule J est la 4-méthyl-5-aminopyrimidine, la 4-amino-5-méthylpyrimidine, la 5-amino-6-méthylpyrimidine ou 1a 5-méthyl-6-aminopyrimidine, chacune facultativement substituée sur le cycle ou groupe méthyle par un substituant compatible avec un alkyllithium.
